# EUROPEAN PATENT SPECIFICATION

(11) **EP 1 334 365 B1**
(45) Date of publication and mention of the grant of the patent: **21.02.2007**
(21) Application number: 00992674.2
(22) Date of filing: 07.12.2000
(51) Int. Cl.: G01N 33/68, C07K 14/51

(54) **SCREENING METHODS FOR BONE MORPHOGENETIC MIMETICS**
METHODEN ZUM SCREENING KNOCHENMORPHOGENETISHEMIMETIKA
PROCEDES DE CRIBLAGE DE MIMETIQUES MORPHOGENETIQUES OSSEUX

(30) Priority: 06.11.2000 US 246231 P
(43) Date of publication of application: 13.08.2003
(73) Proprietor: Thrasos, Inc., Waltham, MA 02453 (US)
(72) Inventor: BOSUKONDA, Dattatreymurty, Shrewsbury, MA 01545-3115 (US)
(74) Representative: Murphy, Colm Damien
(86) International application number: PCT/US2000/042657
(87) International publication number: WO 2002/039118

(56) References cited:
- WO-A-00/61774
- WO-A-95/14778
- US-A- 6 083 690
- ESTEVEZ M ET AL: "The daf-4 gene encodes a bone morphogenetic receptor controlling C. elegans dauer larva development." NATURE, vol. 365, 14 October 1993 (1993-10-14), pages 644-649, XP001064638
- THE C. ELEGANS SEQUENCING CONSORTIUM: "Genome sequence of the nematode C. elegans: a platform for investigating biology" SCIENCE, vol. 282, 11 December 1998 (1998-12-11), pages 2012-2018, XP002195036
- LEE-HOEFLICH S.T. ET AL.: "Activation of LIMK1 by binding to the BMP receptor, BMPRII, regulates BMP-dependent dendritogenesis." EMBO J., vol. 23, no. 24, 2004, pages 4792-4801,

## Description

### BACKGROUND OF THE INVENTION

The bone morphogenetic protein (BMP) family is a conserved group of signaling molecules within the transforming growth factor- β (TGF-β) superfamily. This growth factor superfamily mediates cellular interactions and tissue differentiation during development BMP-7, a member of this superfamily, is a homodimeric glycoprotein with an ability to induce cartilage and bone formation *in vivo*, and enhance recovery from stroke. Therefore, BMP-7 has a wide range of applications in the medical field, including treatment of osteoporosis, Paget's disease and other metabolic bone diseases, neural injury, renal osteodystrophy and cardiac ischemia.

Despite the great potential of BMP-7 as a therapeutic agent, its use as a drug is beset with a number of problems. For example, whole bone morphogenetic protein (BMP) mature domain is a large molecule (approximately 250 amino acids) and possesses solubility problems at physiological pH due to its high hydrophobicity. The whole BMP cannot cross the blood-brain barrier, and the protein requires selective routes of administration. In the case of systemic administrations, the initial BMP protein load has undesirable side effects at therapeutic concentrations. There is a tendency for BMP mature domain at high intravenous doses, to form a bony callus at the injection site.

Consequently, there is a need to identify new compounds which retain the activity of BMP-7 but which overcome these shortcomings. Also needed are assays for identifying such compounds.

### SUMMARY OF THE INVENTION

It has now been found that bone morphogenetic protein binds to fragments of their receptor that consists of only the extracellular domain. For example, the extracellular domain of *daf*-4*,* a bone morphogenetic protein receptor from *C*. *elegans,* readily binds bone morphogenetic proteins such as BMP-2, BMP-4 and BMP-7. Based on this discovery, methods for identifying an agent which modulates the activity of a bone morphogenetic protein are disclosed.

A bone morphogenetic protein receptor ligand, a receptor for bone morphogenetic protein, and an agent to be tested are combined under conditions suitable for binding of the bone morphogenetic protein ligand to the receptor for bone morphogenetic protein. The extent of binding of the ligand to the receptor is then determined. The extent of ligand/ receptor binding in the presence of the test agent is compared with the extent of binding in the absence of the agent to be tested under conditions suitable for binding of the ligand to the receptor for the bone morphogenetic protein. A difference in the extent of binding determined indicates that the agent modulates the activity of bone morphogenetic protein-7. Agents identified by this method can be further assessed in *in vivo* and *in vitro* assays for bone morphogenetic activity. Methods are also described utilizing the *daf*-4 receptor.

Also, disclosed is the use of the identified agents as bone morphogenetic protein mimetics. The invention provides agents identified by the methods described above, as well as compositions comprising the agents, and methods of treating a subject using the agents or compositions. Also contemplated is a high throughput assay for identifying agents that modulate the binding of BMP ligand to the receptor for BMP.

The methods of the invention provide a simple, rapid, convenient means for identifying potential therapeutic BMP-like agents lacking the problems associated with the whole BMP molecule. The methods can be configured as a high throughput assay allowing the screening of numerous compounds and compound libraries for bone morphogenetic activity. Thus, the methods of the invention facilitate identification and development of novel bone morphogenetic mimetics for various medical applications.

### BRIEF DESCRIPTION OF THE DRAWINGS

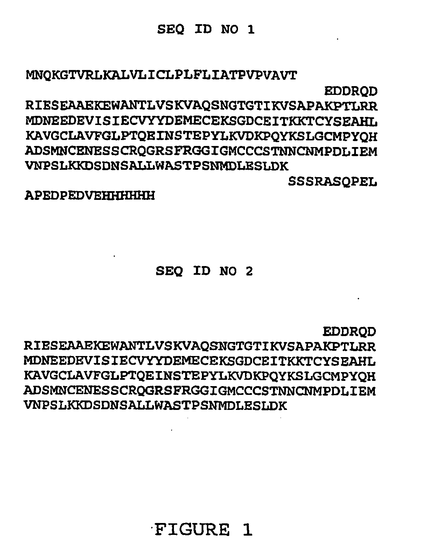

FIG. 1 is the amino-acid sequence of *daf*-4 extracellular protein fragments, SEQ ID NO. 1 and SEQ ID NO 2. SEQ ID NO. 1 includes additional portions on the N and C terminus.
FIG. 2 is a table representing the results from the BMP-peptide activities in the assay described in Example 6.
FIG. 3 is a graphic depiction of the dose-related effects of the BMP peptide F1-2 (SEQ. ID NO 4) on the basal and BMP-7 induced alkaline phosphatase activity in the ROS cell-based assay.
FIG. 4 is a graphic depiction of the dose-related effects of BMP-peptides and unlabeled BMP on the binding of radio-iodinated BMP to ROS cell plasma membranes.
FIG. 5 is a graphic dose-related effect of BMP test agent H2C (SEQ ID NO 6) on the basal and BMP induced alkaline phosphatase activity in the ROS cell-based assay.
FIG. 6 is a graphic depiction of the dose-related effects of the BMP test agent F2-2 (SEQ ID NO.7) on the basal and BMP induced alkaline phophatase activity in the ROS cell-based assay.
FIG. 7 is a graphic depiction of the dose related effects of BMP test agent F2-2 (SEQ ID NO 7) and unlabeled BMP on the binding of radio-iodinated BMP to ROS cell plasma membranes.
FIG. 8 is a graphic depiction of the dose related effect of the BMP test agent F2-3 (SEQ ID NO 9) and of unlabeled BMP on the binding of radioiodinated BMP to ROS cell plasma membrane bound receptors.
FIG. 9 is a graphic depiction of the dose effect of the BMP test agent (SEQ ID NO 9) on the basal and the BMP induce alkaline phosphatase activity in a ROS cell bio-assay.
FIG. 10 is a HPLC profile of purified ECD of *daf-*4 receptor after reversed phase chromatography on a C18 column.

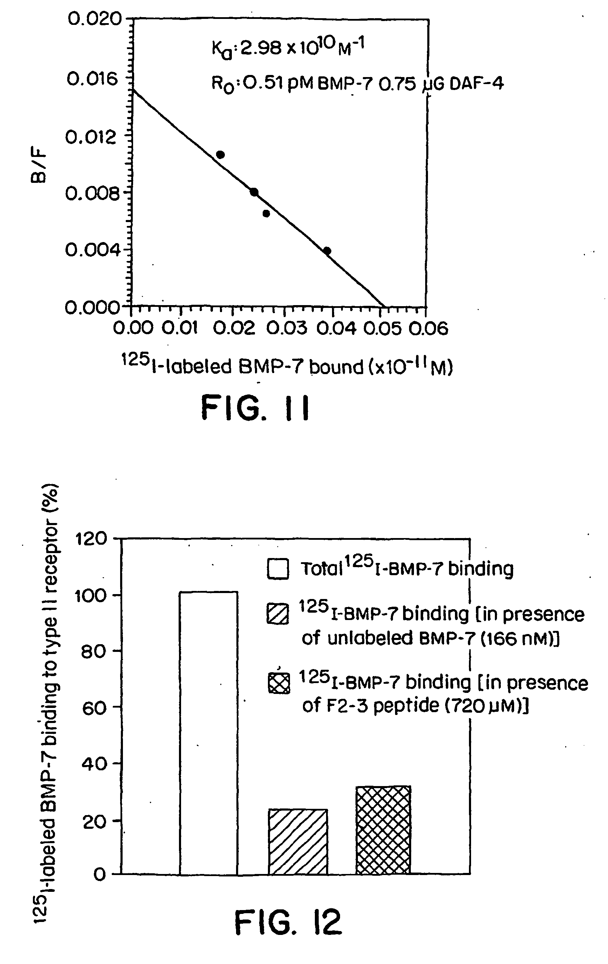

FIG. 11 is a graphic depiction of the affinity of ECD of *daf*-4 receptor for BMP by Scatchard analysis.
FIG. 12 is a graphic depiction demonstrating that F2-3 (SEQ ID. NO 9) binds to ECD of *daf*-4 by a rapid solid phase assay.

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

### DETAILED DESCRIPTION OF THE INVENTION

The invention is based on bone morphogenetic proteins, especially BMP-7, and the interaction of these molecules to their receptor, particularly to the extracellular portion of the receptor, and more particularly the *C. elegans daf*-4 receptor and its extracellular domain (ECD).

Compounds which enhance the activity of BMP-7, e.g., a peptide having the amino acid sequence (agonists) SEQ ID NO. 7 and 9, can be used to treat subjects in whom the activity of BMP-7 provides a useful therapeutic effect. For example, such compounds stimulate the formation of new bone and could therefore be used to treat osteoporosis. BMP-7 has also been shown to stimulate the branching of dendritic trees of neurons and enhance the functional remodeling or remaining neural tissues following neural ischemia such as stroke when used within a therapeutic time window. BMP-7 can also be potentially used to promote recovery of drug induced ischemia in the kidney and the effects of protein overload. There is also evidence that BMP-7 can ameliorate the effects of acute myocardial ischemic injury and reperfusion injury and prevent restenosis after angioplasty. BMP-7 may be useful in the treatment of certain types of cancer, e.g, prostate cancer and pituitary adenomas, and in treating female reproductive disorders by enhancing expression of FSHβ (follicle stimulating hormone- β subunit). In addition, it has been shown the BMP-7 can ameliorate the effects of chemically induced inflammatory lesion in the colon.

Compounds which block the activity of BMP-7 ("BMP-7 antagonists"), e.g., peptides having the amino acid sequence (agonists) SEQ ID NO'S 3, 4, 5 , 6 and 10 can be used to treat subjects in whom a reduction of BMP-7 activity can provide a useful therapeutic effect. Examples include subjects with pituitary abnormalities and other endocriopathies . Also included are subjects in need of treatment with anti-angiogenesis agents (e.g. cancer patient) or anti-arteriosclerotic agents and subjects requiring the prevention of restenosis (e.g. patients following angioplasty).

The methods of this invention detect compounds which block binding between bone morphogenetic protein ligand and the bone morphogenetic receptor. Therefore, it detects both agonists and antagonists. Agonists and antagonists are agents that "modulate" BMP activity. Stimulatory activity can be differentiated from inhibitor activity by additional assays, *in vitro* functional assays or *in vivo* assays. Furthermore, if an agent is found to be active in an assay of the present invention, the agent can be tested in the other assays to further assess the bone morphogenetic activity, such as the assays of Examples 4, 5 and 6. These other assays can be useful to further select the most desirable agents possessing a certain bone morphogenetic activity. Other assays include but are not limited to assessing the agent's ability to restore large diaphyseal segmental bone defect in a rabbit model (Cook *et al.* J Bone and Joint Surg. 76-A: 827-838. 1994); assessing the agent's neuroprotective effect in a rat model of cerebral hypoxia/ischemia (Perides *et al.,* Neuroscience Letters 187: 21-24, (1995)); assessing the agent's ability in preventing damage due to ischemia/reperfusion injury in a rat model (Vukicevic *et al.* J Clin Invest. 102: 202-214. 1998); assessing the tumor-suppressing activity (Wand *et al.,* Abstract No. 30, International Conference of Bone Morphogenetic Proteins, Lake Tahoe, CA (2000)) of the agent in a male rat model of prostate tumor implants (Thalmann *et al.,* Prostate, Vol. 44: 91-103 (2000)). Also the agents can be further tested by examining anti-ischemic and endothelial protective actions of identified bone morphogenetic agents in a rat model of myocardial ischemia induced by ligation of coronary artery (Lefer *et al*., J Mol Cell Cardiol. 24: 585-593 (1992)) and assessing the systemic effects in preventing osteoporosis in aged in an ovariectomized female rat model (Hurtajada-Molleni *et al.,* J. or Endocrinology 165:663-668 (2000)).

The term "bone morphogenetic protein ligand" can encompass a peptide, molecule, protein or other entity that binds to the receptor for bone morphogenetic protein. Preferred "bone morphogenetic proteins" are wild type proteins, including but not limited to bone morphogenetic protein-2, bone morphogenetic protein-4, bone morphogenetic protein-5, bone morphogenetic protein-6, bone morphogenetic protein-7, activin and other growth factors of the TGF-β superfamily or agents that are structurally or functionally equivalent. Other agents such as fragments which bind BMP receptor, such as the test agents described by SEQ ID NO's 3, 4, 5, 6, 7, 9 and 10 are intended to be encompassed by the term "bone morphogenetic protein ligand" as it is used herein.

Bone morphogenetic protein action utilizes a serine-threonine signaling system involving two types of receptors. The Type I receptor is involved in signal transduction and the Type II receptor recognizes the ligand BMP. The current model for receptor activation involves BMP binding to Type II receptor which causes both molecules to undergo conformational changes producing a ligand-receptor complex which is then capable of binding with two monomers of Type I receptor. Following oligomerization and activation, Type II receptor cross phosphorylates Type I receptor to initiate signal transduction and the cascade of intracellular events leading to target cell responsiveness.

As used herein, the receptor is a protein which binds bone morphogenetic protein and can transmit a signal to the nucleus of a cell as a result of such binding or a fragment of the protein which retains bone morphogenetic activity, for example, the extracellular domain of the protein. Preferably, the receptor is a Type II receptor, or a portion of a Type II receptor which binds BMP, such as the extracelluar domain of a Type II receptor. The BMP extracellular domain (ECD) is the portion of the receptor which is outside of the plasma membrane and binds BMP . Optionally, the ECD can include additional amino acids at the C or N terminus which are not in the wild type sequence and which do not affect function, such as a poly histidine chain. In one preferred embodiment, the ECD from *daf*-4 is used in the method of present invention to facilitate purification. For example, the ECD of *daf*-4 can have the amino acid sequence as identified by SEQ ID NO 1 and SEQ ID NO 2. The receptor may have greater than 90% homology to *daf*-4, or more preferably greater that 95% homology to *daf*-4 or a fragment thereof comprising the ECD.

In another embodiment the invention is a method for identifying an agent which modulates the activity of bone morphogenetic protein-7. A bone morphogenetic protein receptor ligand, the extracellular domain of a bone morphogenetic protein, preferably the *daf*-4 receptor, and an agent to be tested are combined under conditions suitable for binding of the ligand to the extracellular domain of *daf*-4 receptor and determining the extent of binding of the ligand to the extracellular domain of *daf*-4 receptor; and comparing the extent of binding in the absence of the agent to be tested under conditions suitable for binding of the ligand to the extracellular domain of *daf*-4 receptor, wherein a difference in the extent of binding determined in the presence of the test agent from the binding determined in the absence of test agent indicates that the agent modulates the activity of bone morphogenetic protein-7. The agents identified may be further assessed for bone morphogenetic activity in other *in vitro* or *in vivo* assays to assess bone morphogenetic protein activity.

In a further embodiment, bone morphogenetic protein is labeled, preferably with a radioisotope.

In another embodiment the receptor is a polypeptide having the amino acid sequence of SEQ ID NO. 1 or SEQ ID NO. 2.

In still another embodiment, the invention is a polypeptide analog of *daf*-4, wherein the analog comprises a polypeptide having the amino acid sequence of SEQ ID NO. 1 of SEQ ID NO.2. The polypeptide binds to a bone morphogenetic protein but does not bind with type I receptors and/or does not elicit signal transduction. In still another invention the polypeptide has the amino acid sequence of SEQ ID NO. 1 or SEQ ID NO. 2.

Another embodiment of the present invention is an agent identified by the methods disclosed. Examples include polypeptides having the amino acid sequence of SEQ ID NO. 3, 4, 5, 6, 7, 9 and 10 and physiologically salts thereof.

Another embodiment of the present invention is a pharmaceutical composition comprising an agent identified by the methods of this invention, including polypeptides represented by SEQ ID NO. 3, 4, 5, 6, 7, 9 and 10, and a pharmaceutically acceptable carrier.

Still another embodiment is a method for treating a subject with osteoporosis, metabolic bone disease, Paget's disease, neurologic stroke, renal failure, cardiac ischemia, cancer, infertility, pituitary abnormalities and other endocriopathies, inflammatory bowel disease by administering an effective amount of the pharmaceutical composition or the agent i.e., an agent that enhances BMP activity, such as a polypeptide having the amino acid sequence of SEQ ID NO 7 or 9 to the subject. Also included are methods for treating subjects in need of treatment with anti-angiogenesis agents (e.g. cancer patient) or anti-arteriosclerotic agents and subjects requiring prevention of restenosis (e.g. patients following angioplasty), by administering an agent that blocks BMP activity, such as a polypeptide having the amino acid sequence of SEQ ID NO. 3, 4, 5, 6, or 10.

In still a further embodiment the invention is a high throughput screening assay for identifying an agent which modulates the activity of bone morphogenetic protein-7. Radiolabled bone morphogenetic protein-7, the extracellular domain of *daf*-4 and a test agent, are combined under conditions suitable for binding of bone morphogenetic protein-7 to the extracellular domain of *daf*-4. The extent of binding of bone morphogenetic protein-7 to the extracellular domain of *daf*-4 is determined and compared the extent of binding determined in the absence of the test agent under conditions suitable for binding of bone morphogenetic protein-7 to the extracellular domain of *daf*-4 receptor. A difference in the extent of binding indicates that the agent modulates the activity of bone morphogenetic protein-7. Preferably, the ECD is coupled to a solid phase such as a PVDF membrane. The immobilized receptor characterizes a receptor or ligand which is coupled to a solid phase via an interaction, typically a chemical reaction for instance the hydrophobic interaction of the receptor to the PVDF membrane.

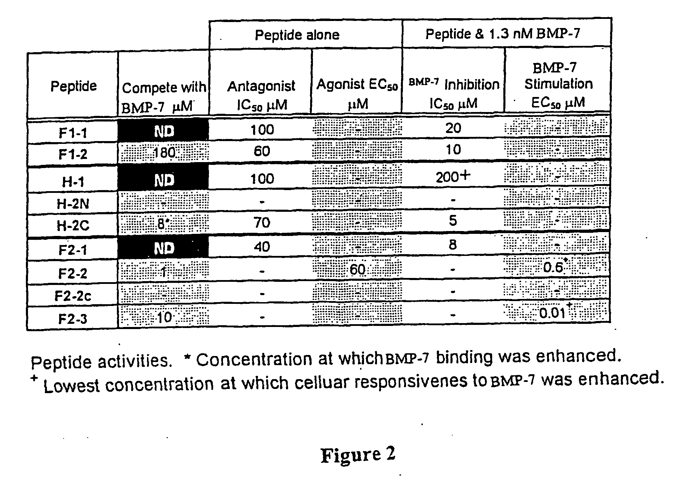

In a typical experiment, a control is run either simultaneously or staggered. The control determines the extent of binding in the absence of test compound i.e. omitting the test compound or replacing it with a control compound to determine non-specific binding. For example, a control compound for the active peptides identified in the Examples was a peptide consisting of the sequence of amino acids in reverse order, identified in Fig 2 has having no activity in Examples 1, 2 or 3. The term "extent of binding" is the amount of activity obtained by quantifying binding by measuring for instance a radiolabel or colormetric agent (e.g. dye), or other standard method used in the art.

The methods of the invention may be designed as standard competition assays well known in the art. The receptor or BMP can be coupled or immobilized to a solid phase (e.g., filter, membrane such as PVDF, cellulose or nitrocellulose), plastic (e.g., microtiter plate, dipstick), glass (e.g., slide), bead (e.g., latex beads), particle, organic resin, or other organic or non-organic solid phase) or a fluid (e.g., TRIS buffer or phosphate buffer) phase. Coupling the BMP or receptor to the solid or fluid phase can be accomplished by standard methods, including, for example, air drying or chemical reaction. The binding of receptor to ligand can be optimized by varying other conditions such as pH (physiological), temperature (4°C to 37°C), buffer, incubation time and concentration. Suitable conditions are at physiological pH and temperature, using 50 mM HEPES buffer.

The peptides identified in the Examples as having bone morphogenetic activity can possess a sufficiently acidic, a sufficiently basic, or both functional groups, and accordingly react with any of a number of inorganic bases, and inorganic and organic acids, to form a salt. Acids commonly employed to form acid addition salts are inorganic acids such as hydrochloric acid, hydrobromic acid, hydroiodic acid, sulfuric acid, phosphoric acid, and the like, and organic acids such as p-toluehesulfonic acid, methanesulfonic acid, oxalic acid, p-bromophenyl-sulfonic acid, carbonic acid, succinic acid, citric acid, benzoic acid, acetic acid, and the like.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like.

An "effective amount" of the peptides of the present invention is the quantity of peptide which results in a desired therapeutic and/or prophylactic effect while without causing unacceptable side-effects when administered to a subject having one of the aforementioned diseases or conditions. A "desired therapeutic effect" includes one or more of the following: 1) an amelioration of the symptom(s) associated with the disease or condition; 2) a delay in the onset of symptoms associated with the disease or condition; 3) increased longevity compared with the absence of the treatment; and 4) greater quality of life compared with the absence of the treatment.

An "effective amount" of the peptide administered to a subject will also depend on the type and severity of the disease and on the characteristics of the subject, such as general health, age, sex, body weight and tolerance to drugs. The skilled artisan will be able to determine appropriate dosages depending on these and other factors. Typically, an effective amount of a peptide of the invention can range from about 0.01 mg per day to about 1000 mg per day for an adult. Preferably, the dosage ranges from about 0.1 mg per day to about 100 mg per day, more preferably from about 1.0 mg/day to about 10 mg/day.

The peptides of the present invention can, for example, be administered orally, by nasal administration, inhalation or parenterally. Parenteral administration can include, for example, systemic administration, such as by intramuscular, intravenous, subcutaneous, or intraperitoneal injection. The peptides can be administered to the subject in conjunction with an acceptable pharmaceutical carrier, diluent or excipient as part of a pharmaceutical composition for treating the diseases discussed above. Suitable pharmaceutical carriers may contain inert ingredients which do not interact with the peptide or peptide derivative. Standard pharmaceutical formulation techniques may be employed such as those described in Remington's Pharmaceutical Sciences, Mack Publishing Company, Easton, PA. Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Some examples of suitable excipients include lactose, dextrose, sucrose, trehalose, sorbitol, and mannitol.

A "subject" is a mammal, preferably a human, but can also be an animal, e.g., domestic animals (e.g., dogs, cats, and the like), farm animals (e.g., cows, sheep, pigs, horses, and the like) and laboratory animals (e.g., rats, mice, guinea pigs, and the like).

### EXAMPLES

### Peptides

The bold indicates these cysteine residues are involved in a disulfide bond.
F1-1 (CELYVSFRDLGWQDWIIAPEGYAAYC SEQ I.D NO.3)
F1-2 (CFRDLGWQDWIIAPC, SEQ I.D NO.4)
H-1 (CAFPLNSYMNATNHAIVQTLVHFINPETVPKC SEQ ID NO 5.)
H-2C (CCFINPETVCC, SEQ I.D NO.6)
F2-2 (CYFDDSSNVIC SEQ I.D NO.7)
F2-2C (CIVNSSDDFYC SEQ I.D NO.8)
F2-3 (CYFDDSSNVICKKYRS, SEQ I.D NO.9)
F2-1 (CLNAISVLYFDDSSNVILKKYRNMWRC, SEQ ID NO. 10)

### Example 1

*Radio-ligand receptor Assay:* The receptor-binding activities of various BMP ligands (New York Public Health Labs, Abany, NY) were determined by an equilibrium displacement binding isotherm assay using BMP-7 receptor-enriched plasma membrane fraction of ROS cells (ATCC 17-2.8) and ¹²⁵I-labeled BMP-7 (NEN, Billerica, MA) as ligand. Purified BMP-7 was radio-iodinated BMP-7 to a specific activity of 70-78 uCi/ug by a modified procedure of lactoperoxidase method (Schneyer, A.L., *et al.,* Endocrinology 119, 1446-1453 (1986)). The percent bindability of radioiodinated BMP-7 to excess receptor is 30-37%. Receptors from ROS cells are obtained by a procedure previously described (Dattatreyamurty, B., *et al.,* (1986) J. Biol. Chem. 261.). These preparations contain a single class of BMP-7 binding sites with an affinity (Kₐ) of 4.38 x 10⁹ M⁻¹ and an average BMP-7 binding capacity of 3.6 pmol/mg protein. In a typical assay, fixed amounts of ¹²⁵I-labeled sol. BMP-7 (-80.000 cpm) were incubated with a fixed amount of receptor-enriched plasma membrane fraction in the presence (5ug. for non-specific binding) or absence of excess unlabeled BMP-7 (for total binding). Displacement curves were generated with increasing concentrations of BMP-7 standard preparation (5-2000 ng). The assay incubations were carried out in a reaction volume of 300 µl with shaking for 22 hrs. at 4° C. Bound and unbound ¹²⁵I-labeled BMP-7 were separated by centrifugation (30,500 x g. 30 minutes). The supernatants were aspirated and pellets washed before counting in a gamma counter (Packard Instr. Downers Grove, IL). The concentration of test agent required to give 50% inhibition of total specific binding of ¹²⁵I-labeled BMP-7 (ED₅₀) was calculated from competitive data.

### Example 2

*Type II Receptor-based ligand-blot technique:* A novel ligand blot method was developed to characterize BMP-7 and test agent binding to ROS cell receptor. Test agents were analyzed by this method to determine their ability to interact with type II receptor. Based on their ability to inhibit ¹²⁵I-labeled BMP-7 binding to the receptor. In a typical experiment, receptor-enriched ROS cell plasma membrane fraction was treated with SDS (final concentration 1.6% w/v) in the presence of 17% glycerol on ice, as described previously (Dattatreyamurty, B. & Reichert Jr., L.E. (1992) Endocrinology 131, 2437-2445.) and subjected to SDS-PAGE under nonreducing conditions and without prior heating of the samples, according to the procedure of Laemmli (Laemmli, UK (1970) Nature 227, 680-685). Receptor samples were electrophoressed at 35 mA and 4°C. in gradient (5-8% or 5-10% w/v) acrylamide separating gels. Pre-stained markers of known molecular weights were used as standards. After SDS-PAGE, the resolved proteins from acrylamide gels were transblotted onto PVDF membranes (Immobilon-P transfer membranes) using an Pharmacia-LKB 2005 Transphor Unit at constant current (0.2 A) and 4°C. for 16 h. Ligand blotting was carried out as follows. Briefly, the sample lanes were incubated with blocking buffer (3% BSA in 50 mM HEPES buffer, pH 7.4) overnight in cold (4°C), and further incubated in 50 mM HEPES buffer, pH 7.4 containing 0.5% BSA, 10 mM MgCl₂, 1 mM CaCl₂ and ¹²⁵I-labeled BMP-7 (400,000 cpm/ml) in the absence or presence of excess unlabeled BMP-7. Receptor containing lanes were also incubated with ¹²⁵I-labeled BMP-7 in the presence of test agents to determine receptor-binding properties of the test agents The blots were rinsed three times with 50 mM HEPES buffer, pH 7.4 containing 5 mM MgCl₂ air-dried and subjected to autoradiography. The results indicate the receptor binding activity of test agent as determined by its ability to inhibit the binding of radiolabeled BMP-7 to ROS cell type II BMP receptor.

### Example 3

### In vitro bioassays

*ROS Cell Based Alkaline Phosphatase Assays:* BMP-7 and test agents that bind BMP-7 receptors were analyzed by *in vitro* bioassay. This assay defines the role of these test agents at the receptor-binding site. It is believed that some of the test agents can effectively interact with receptors and inhibit BMP-7 induced target cell responsiveness, thereby acting as functional antagonists. Alternatively, these agents may mimic BMP-7 functions inducing alkaline phosphatase activity in ROS cells, thus acting as functional agonists. An assay procedure as described by Maliakal, J.C., *et al.,* Growth Factors 11, 227-234, (1994) determined the biological activities of BMP-7 peptides. In a typical experiment, rat Osteosarcoma (17/2.8) cells were plated in 96 well plates (3.0 x 104 cells/well) and incubated overnight at 37 C in 5-6% CO₂ incubator. Next day, the plates were examined to make sure that cells are healthy & confluent. Cells were treated with increasing concentrations of BMP-7 standard (1-10,000 ng/ml) or test agents (0.02-200 µM) or appropriate concentrations of test agent alone or with BMP-7 standard prepared in medium containing 1% FBS and incubated for 2 days at 37 C in 5-6% CO₂ incubator. The cellular content of alkaline phosphatase activity was determined by the method of Reddi, A.H. & Huggins, C.B. Proc. Natl. Acad. Sci. USA 69, 1601-1605 (1972). Enzyme estimations were routinely carried out in 96 well plates. Following removal of culture medium, cells were washed with pre-warmed PBS (150 µl) and further incubated in 100 µl of pre-warmed 1% Triton X-100 for 30 minutes at 37 C. Plates were centrifuged for 10 minutes at full speed, and recovered samples (each 15 µl) were assayed for enzyme activity by adding 90 µl p-nitrophenyl phosphate (Sigma, St. Louis, MO) as a substrate in 0.05 M glycine-NaOH buffer, pH 9.3 and incubating for 20 minutes at 37 C. The reaction was stopped by adding 75 µl of 0.2 N NaOH/well and absorbance at 405/490 nm is measured on a Dynatech MR 700 plate reader( Dynatech Laboratories, U.K.). Results are expressed as concentration of peptide/small molecule inhibitor (with antagonist activity) required to give 50% inhibition of maximum response to BMP-7 standard. The activities of test agents that stimulate cell responsiveness (with agonist activity), are expressed relative to BMP-7 standard.

### Results:Activity profiles of BMP test agents:

F1-1 (SEQ ID NO. 3) has cyclized ends When ROS cells were incubated with increasing concentrations of this peptide (2 to 260 µM) together with BMP-7 (1.33 nM), the peptide behaved as an antagonist and inhibited BMP-7 induced cellular responsiveness in a dose-dependent manner. A 50% inhibition of cellular responsiveness to BMP-7 was observed at a peptide concentration of 20 µM. F1-1 alone, was effective only at higher concentration (52-260 µM) to inhibit the basal cellular responsiveness (Fig. 2).

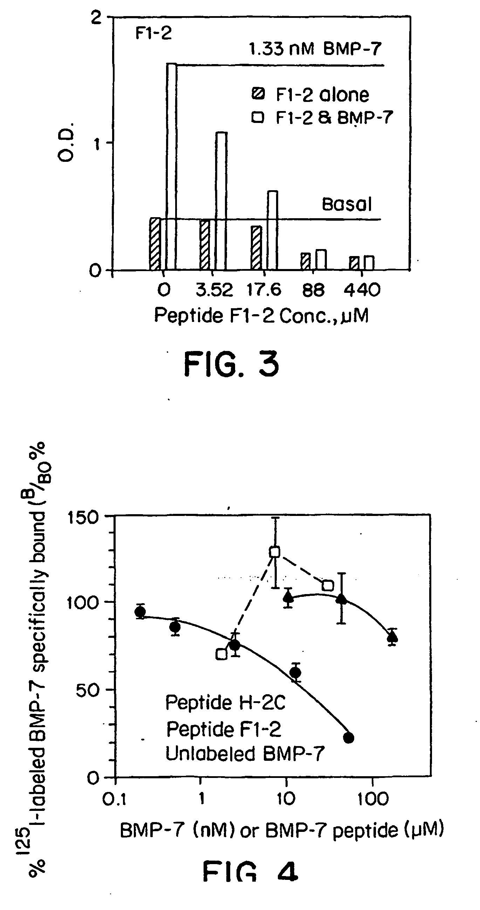

Peptide F1-2 (SEQ ID NO. 4) is a 15 residue peptide F1-1 that is cyclized at the ends In the ROS cell based bio-assay, the peptide behaved similar to F1-1, weakly inhibiting the basal cell responsiveness. When the peptide was tested together with BMP-7, it behaved as an antagonist and inhibited the cell responsiveness to BMP-7 in a dose-dependent manner (Fig. 3). A 50% inhibition of the cell responsive to BMP-7 was observed at a peptide concentration of 10 µM. When this peptide was tested in a radioligand receptor assay, it required relatively higher concentrations (179 µM) to inhibit the binding of ¹²⁵I-labeled BMP-7 to the receptor, thus showing a relatively weak activity in the assay (Fig. 4).

Heal peptides: Peptide H-1 (SEQ ID NO. 5) is cyclized. In the ROS cell based bio-assay, this peptide behaved as a weak antagonist as it required relatively high concentrations (44-220 µM) to inhibit the BMP-7 induced cell response. Peptide alone was also effective only at very high concentrations in inhibiting the basal alkaline phosphatase activity of the ROS cell (Fig. 2).

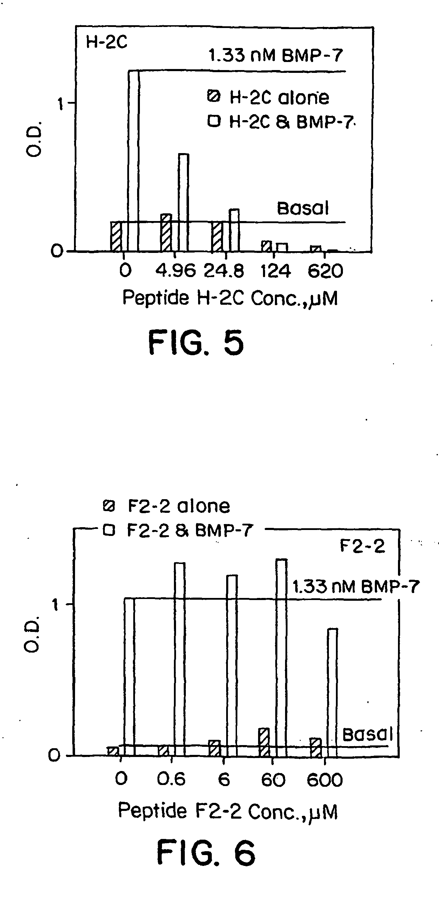

Peptide H-2C (SEQ ID NO. 6) is cyclized. In the radioligand receptor assay, this peptide at 8 µM concentration potentiated the binding of ¹²⁵I-labeled BMP-7 to the receptor (Fig. 4). In ROS cell based bio-assay, however, the peptide behaved as an antagonist inhibiting the basal as well as the BMP-7 induced cell response. A 50% inhibition of cellular responsiveness to BMP-7 was observed at a peptide concentration of 5 µM (Fig. 5).

Peptide F2-1 (SEQ ID NO. 10) is cyclized. In the ROS cell-based bio-assay this peptide effectively inhibited both the basal as well as the BMP-7 induced cellular responsiveness in a dose-dependent manner. The peptide, at a concentration of 8 µM, inhibited the BMP-7 induced cell response by 50% (Fig. 2).

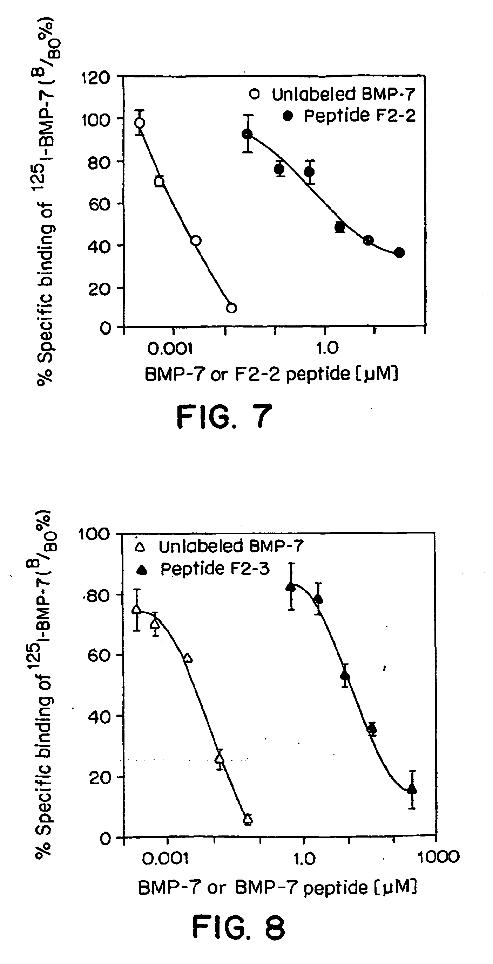

Peptide F2-2 (SEQ ID NO. 7) contains 9 residues its ends are cyclized. In the ROS cell based bio-assay, the peptide behaved as a weak agonist at lower concentrations (0.6-60 µM), as it slightly potentiated (20%) BMP-7 induced cell responsiveness (Fig. 6). At higher concentrations, however, the peptide inhibited the responsiveness to BMP-7. When this peptide was tested in a radioligand receptor assay, it effectively inhibited the binding of ¹²⁵I-labeled BMP-7 in a dose-dependent manner with an ED₅₀ of 1 µM (Fig.7).

Peptide F2-2c (SEQ ID NO. 8) served as a control peptide in the assay. This is a cyclized peptide having 9 residues identical to those of peptide F2-2, but in a reversible order. In radioligand receptor assay, this peptide was inactive. Also, the peptide has no effect on basal or BMP-7 induced ROS cell responsiveness.

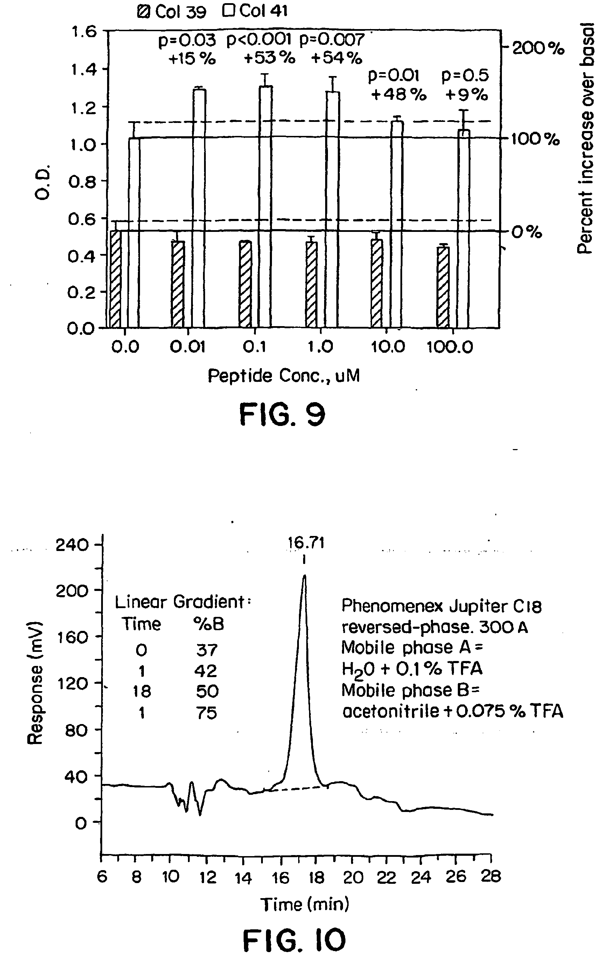

Peptide F2-3 (SEQ ID NO. 9) contains 16 residues that are identical to that of F2-2 and a 5 residue C-terminal extension. The peptide is cyclized into an 11 residue loop by an internal Cys replacement plus an N-terminus Cys. It contains 2 negative charges in the finger 2 loop and 3 positive charges in the C-terminus of the tip. In the radioligand receptor assay, the peptide gave a dose-related inhibition of ¹²⁵I-labeled BMP-7 binding to the receptor, with an ED₅₀ of 10 µM (Fig. 8). Moreover, the calculated slopes of the dose-response lines for the peptide and a reference preparation unlabeled BMP-7 were similar suggesting that the dose-response lines were parallel. In the ROS cell-based bio-assay, the peptide had no significant effect on basal cell responsiveness, but behaved in an agonistic manner in the presence of a sub-maximal concentration of BMP-7 (1.33 nM). Interestingly, the peptide was extremely effective at low concentrations (0.01 - 1 µM) where it significantly potentiated BMP-7 induced cellular alkaline phosphatase activity (Fig. 9). For peptide concentrations as low as 0.01, 0.1 and 1 µM, the percent increases in BMP-7 response observed (relative to the absence of peptide) were 53% (P<0.001), 54% (P=0.007) and 48% (P=0.01), respectively.

### Example 4

*Assay of dendritic growth in rat sympathetic neurons:* This assay is based on the ability of BMP-7 to specifically induce dendritic growth in perinatal rat sympathetic neurons. The assay was set up as described in Lein P. *et al.,* Neuron 15(3), 597-605, (1985). In brief, sympathetic neurons were dissociated from the superior cervical ganglia of perinatal rats (Holtzman rat fetuses of 20-21 day pregnancy) according to the method of Higgins, D., Lein, P., Osterhout, D. & Johnson, M.I. (1991). In Culturing Nerve Cells. G. Bonker and K. Goslin, eds. (Cambridge, Mass.: MIT Press) pp. 177-205. The cells were then plated at low density (~10 cells/mm2) onto polylysine-coated (100 ug/ml) coverslips and maintained in a serum-free medium containing beta-NGF (100 ng/ml). Non-neuron cells were eliminated by treatment with an antimitotic agent (cytosine-beta-D-arabompfirampsode. 1 uM) on days 2 and 3. One to 2 days were then allowed to lapse for recovery before beginning experimental treatment. Beginning on day 5, the medium of cultures was continuously supplemented with BMP-7 (50 ng/ml) or varying concentrations of test agent or BMP-7 and test agent for 5 days and then immunostained with a dendrite specific antibody (SM132, and antibody to nonphosphorylated forms of the M and H neurofilament subunits, Sternberger Monoclanals, Inc.). Cellular morphology was analyzed by fluorescence microscopy. Results were be presented as the number of dendrites per cell. Neurons in control cultures typically have only 1 process, a long axon, while neurons exposed to BMP-7 are multipolar, having several tapered dendrites and 1 axon.

### Example 5

### In vivo bioassay

Subcutaneous implantation assay in rodents: This assay is based on the ability of BMP-7 to induce bone formation in the rat. A procedure previously as described in detail in Sampath, T.K. *et al*., (1992) J.Biol. Chem. 267, 20352-20362.was followed to determine *in vivo* effect of test agents that showed activity in *in vitro* assays as described above. Briefly, 1.2 mg of bovine bone matrix (collagen carrier)was added to BMP-7 or test peptide or small molecule or BMP-7 and test peptide in 200 µl of 50% acetonitrile, 0.15 control. A minimum of 6 animals per group were used. The day of implantation is designated as day 0 of the assay. Implants were removed on day 14 for evaluation. Bone-forming activity in the implants was monitored by the calcium content essentially as described by Reddi, A.H. & Huggins, C.B. (1972) PNAS USA 69, 1601-1605. For histology, implants were fixed in Bouin's solution, embedded in JB4 plastic medium, cut into 1 um sections, and stained by toluidine blue. The specific bone-forming activity was expressed as the amount of test peptide/small molecule required to exhibit half-maximal bone forming activity. BMP-7 induced bone formation by day 14. (Data not shown)

### Example 6

### Type II Receptor-based high throughput screening (HTS) assay:

*Preparation of type II Receptor ECD:* Highly purified extracellular domain (ECD) of the type II receptor *daf*-4 was used in this assay. The ECD of the *daf*-4 receptor was cloned and vectors( pCMV5 expression vectors, *daf*-4 sequence including the mammalian consensus start site and the hexa-histidine sequence tag) are constructed for the expression of this molecule in mammalian cells. In this construct, the ECD has been linked to a hexa-Histadine sequence tag for subsequent purification and detection. The transfected DHFR(-) CHO cell line PJ511.9.8 has been used for the *daf*-4 expression as outlined below. This cloning method can also be utilized with other bone morphogenetic proteins.

*Cell culture production of large quantities (3.5L*/*week) of daf4:* A cell bank of this clone has been established and from this bank, one vial of cells is used to establish a scale up train for eventual micro carrier seeding. Initial scale up is done in 150cm2 T-flasks followed by cellular expansion in 850cm2 roller bottles. The harvested cells were allowed to settle on cytodex micro carrier beads and the growth phase of cells is continued in a controlled 7L bioreactor setting, until microscopic observation indicates that bead confluency is greater than 80%. During growth phase, media changes were done every 4^{th} or 5^{th} day. Following the growth phase, further media changes were done using alternate basal formulations or media containing reduced serum and a protease inhibitor aprotinin, to facilitate protein production in a medium more suitable for subsequent protein purification. Media changes and subsequent conditioned media harvests were done every 4-7 days, depending on the quality and quantity of the product. Conditioned media harvests are clarified by centrifugation followed by filtration through a membrane (0.2 um). Micro carrier systems utilized in this manner have lasted more than one month from initiation, and have supplied over 30 liters of conditioned media.

*Purification of daf-4 ECD:* A purification protocol to process large volumes of conditioned media (3.5 L/week) was developed. The procedure involves initial isolation of *daf*-4 ECD by using Talon (Pharmacia Tech.) Immobilized metal affinity column. As *daf*-4 ECD protein is linked to a hexa-His sequence, the protein is bound to the metal affinity column and is subsequently eluted by imidazoic (120 mM) containing buffer. The pooled fractions from the metal affinity column were further purified by gel filtration chromatography on Sephacryl S-200 column to remove contaminating components of very high molecular weight. The active protein eluted from Sephacryl column was purified by reversed phase HPLC using C18 column with a linear gradient of 20% to 90% of acetonitrile and 0.075% TFA. An estimated yield of highly purified daf-4 ECD from each batch of 3.5L conditioned medium is approximately 1.5 to 2 mg. The purified preparation was analyzed by reversed phase HPLC. A chromatographic profile of the preparation showed a single peak. A novel ligand-blot techniquewas developed to evaluate the binding of BMP-7 to the purified *daf*-4 ECD. Upon ligand blotting with ¹²⁵I-labeled BMP-7, the ligand effectively bound to *daf*-4 ECD and identified two closely migrated bands. *daf*-4 ECD is heavily glycosylated, and differences in the carbohydrate composition of its components may contribute to the observed molecular heterogeneity of the *daf*-4 ECD preparations. Moreover, the observed ¹²⁵I-labeled BMP-7 binding to *daf*-4 was inhibited by the excess unlabeled BMP-7 indicating a high degree of specificity with which *daf*-4 ECD binds BMP-7. Scatchard analysis of quantitative BMP-7 binding data indicated that the ECD of *daf*-4 contains a single class of high affinity binding sites for BMP-7 with an association constant (Kₐ) of 2.96 x 10¹⁰ M⁻¹ and a binding capacity of 0.51 pM per 750 ng *daf*-4).

HTS assay procedure: BMP-7 binding activity was determined by a rapid solid-phase assay using ¹²⁵I-labeled BMP-7 as ligand and highly purified ECD of *daf*-4 as type II receptor. In a typical assay, ~1 ug of ECD of *daf*-4 receptor (R) is dissolved in 20 ul of 50 mM PBSA, pH 7.4 and immobilized on PVDF membranes (Immobilon-P) by using a slot-blot apparatus (VacuSlot-VS). A similarly immobilized polyclonal anti BMP-7 antibody (diluted Ab) has served as positive control, while bovine serum albumin (globulin free) and unrelated receptor ECD have served as negative controls in this assay. The receptor-containing membranes are incubated in 50 mM HEPES buffer, pH 7.4 containing 3% bovine serum albumin (BSA) for 14 h in cold (4 C), to block excess protein binding sites on the membrane. The blocking buffer is the replaced with 50 mM HEPES, ph 7.4 containing 0.5% BSA, 10 mM MgCl₂ and 1 mM CaCl₂. The receptor-containing membranes are further incubated with ¹²⁵I-labeled BMP-7 (-400.000 cpm/ml) in the absence (total binding) or presence (NSB) of excess unlabeled BMP-7 or increasing concentrations of BMP-7 or test peptide/small molecule for 18 h. with slow shaking at room temperature. Finally, the membranes are washed four times with 50 mM HEPES buffer, pH 7.4 containing 5 mM MgCl² and 1 mM CaCl₂, air dried and kept for autoradiography overnight or punched to count the sample-containing membrane segments in an auto-gamma counter. The concentration of test agent/ molecule required to give 50% inhibition of total specific binding of ¹²⁵I-labeled BMP-7 (ED₅₀) will be calculated from competitive data. The results indicated that both unlabeled BMP-7 and BMP-7 peptide (F2-3, SEQ ID NO 9) effectively inhibited (78% and 75% respectively) ¹²⁵I-labeled BMP-7 binding to the ECD of *daf*-4 receptor.

## Claims

1. A method for identifying an agent which modulates the activity of bone morphogenetic protein-7, said method comprising the steps of:
(a) combining a bone morphogenetic protein, receptor ligand, A type II receptor for bone morphogenetic protein and an agent to be tested under conditions suitable for binding of the ligand to the receptor and determining the extent of binding of the ligand to the receptor; and
(b) comparing the extent of binding determined in step (a) with the extent of binding in the absence of the agent to be tested under conditions suitable for binding of the ligand to the receptor. Wherein a difference in the extent of binding determined in step (a) from the binding determined in step (b) indicates that the agent modulates the activity of bone morphogenetic protein 7.

2. A method according to Claim 1, wherein the ligand is labelled, for example with a radioisotope.

3. A method according to Claim 1, wherein the wherein the bone morphogenetic protein is selected from the group consisting of bone morphogenetic protein-2 bone morphogenetic protein-4, bone morphogenetic protein-5, bone morphogenetic protein-6, activin and bone morphogenetic protein-7.

4. A method according to claim 1, wherein the receptor is *daf*-4.

5. A method for identifying an agent which modulates the activity of bone morphogenetic protein-7, said method comprising the steps of:
(a) combining a bone morphogenetic protein receptor ligand, the extracellular domain of a bone morphogenetic protein type II receptor, and an agent to be tested, under conditions suitable for binding of the ligand to the extracellular domain of the receptor and determining the extent of binding of the ligand to the extracellular domain of the receptor; and
(b) comparing the extent of binding determined in step (a) with the extent of activity in the absence of the agent to be tested under conditions suitable for binding of bone morphogenetic protein to the extracellular domain of the receptor,
wherein a difference in the extent of binding determined in step (a) from the binding determined in step (b) indicates that the agent modulates the activity of bone morphogenetic protein-7.

6. A method for identifying an agent which modulates the activity of bone morphogenetic protein-7, said method comprising the steps of:
(a) combining a bone morphogenetic protein receptor ligand, the extracellular domain of *daf*-4 receptor, and an agent to be tested, under conditions suitable for binding of the ligand to the extracellular domain of *daf*-4 receptor and determining the extent of activity of the ligand to the extracellular domain of *daf-*4 receptor; and
(b) comparing the extent of binding determined in step (a) with the extent of activity in the absence of the agent to be tested under conditions suitable for binding of bone morphogenetic protein to the extracellular domain of *daf*-4 receptor. Wherein a difference in the extent of binding determined in step (a) from the binding determined in step (b) indicates that the agent modulates the activity of bone morphogenetic protein-7.

7. A method according to Claim 6, wherein the ligand is labelled, for example with a radioisotope.

8. A method according to claim 6, wherein the bone morphogenetic protein is selected from the group consisting of: bone morphogenetic protein-2, bone morphogenetic protein-4, bone morphogenatic protein-5, bone morphogenetic protein 6, activin and bone morphogenetic protein-7.

9. A method according to Claim 7, wherein the receptor comprises a polypeptide having the amino acid sequence of SEQ ID NO. 1. or SEQ ID NO. 2.

10. A method for identifying an agent which modulates the activity of bone morphogenetic protein-7 comprising:
(a) combining radiolabled bone morphogenetic protein 7, the extracellular domain of *daf*-4, and a test agent, under conditions suitable for binding of bone morphogenetic protein to the extracellular domain of *daf*-4 and determining the extent of binding of bone morphogenetic protein-7 to the extracellular domain of *daf*-4 ; and
(b) comparing the extent of binding determined in (a) with the extent of binding in the absence of the test agent under conditions suitable for binding of bone morphogenetic protein to the extracellular domain of *daf*-4 receptor;
wherein a difference in the extent of binding in (a) from the binding determined in (b) indicates that the agent modulates the activity of bone morphogenetic protein-7.

11. A method of Claim 10, wherein the extracellular domain has the amino acid sequence of SEQ ID NO 1 or 2.

12. A method of Claim 10, wherein the extracellular domain is immobilized on a solid support or PVDF membrane.

## Patentansprüche

1. Verfahren zum Identifizieren eines Mittels, das die Aktivität von Knochenmorphogenetischem Protein-7 moduliert, wobei das Verfahren die Schritte umfasst:
(a) Kombinieren eines knochenmorphogenetischen Protein-Rezeptorliganden, eines Typ II Rezeptors für Knochenmorphogenetisches Protein und eines zu untersuchenden Mittels unter Bedingungen, die für die Bindung des Liganden an den Rezeptor geeignet sind, und Bestimmen des Ausmaßes der Bindung des Liganden an den Rezeptor; und
(b) Vergleichen des Ausmaßes der in Schritt (a) bestimmten Bindung mit dem Ausmaß der Bindung in Abwesenheit des zu untersuchenden Mittels, unter Bedingungen, die für die Bindung des Liganden an den Rezeptor geeignet sind,
wobei ein Unterschied des Ausmaßes der in Schritt (a) bestimmten Bindung zu der in Schritt (b) bestimmten Bindung anzeigt, dass das Mittel die Aktivität von Knochenmorphogenetischem Protein-7 moduliert.

2. Verfahren nach Anspruch 1, worin der Ligand markiert ist, beispielsweise mit einem Radioisotop.

3. Verfahren nach Anspruch 1, worin das Knochenmorphogenetische Protein ausgewählt ist aus der Gruppe bestehend aus Knochenmorphogenetischem Protein-2, Knochenmorphogenetischem Protein-4, Knochenmorphogenetischem Protein-5, Knochenmorphogenetischem Protein-6, Activin und Knochenmorphogenetischem Protein-7.

4. Verfahren nach Anspruch 1, worin der Rezeptor *daf*-4 ist.

5. Verfahren zum Identifizieren eines Mittels, das die Aktivität von Knochenmorphogenetischem Protein-7 moduliert, wobei das Verfahren die Schritte umfasst:
(a) Kombinieren eines knochenmorphogenetischen Protein Rezeptorliganden, der extrazellulären Domäne eines Knochenmorphogenetischen Protein Typ II Rezeptors und eines zu untersuchenden Mittel unter Bedingungen, die für die Bindung des Liganden an die extrazelluläre Domäne des Rezeptors geeignet sind und Bestimmen des Ausmaßes der Bindung des Liganden an die extrazelluläre Domäne des Rezeptors; und
(b) Vergleichen des Ausmaßes der in Schritt (a) bestimmten Bindung mit dem Ausmaß der Aktivität in Abwesenheit des zu untersuchenden Mittels, unter Bedingungen, die für die Bindung von Knochenmorphogenetischem Protein an die extrazelluläre Domäne des Rezeptors geeignet sind,
wobei ein Unterschied in dem Ausmaß der in Schritt (a) bestimmten Bindung zu der in Schritt (b) bestimmten Bindung anzeigt, dass das Mittel die Aktivität von Knochenmorphogenetischem Protein-7 moduliert.

6. Verfahren zum Identifizieren eines Mittels, das die Aktivität von Knochenmorphogenetischem Protein-7 moduliert, wobei das Verfahren die Schritte umfasst:
(a) Kombinieren eines knochenmorphogenetischen Protein Rezeptorliganden, der extrazellulären Domäne des *daf*-4 Rezeptors und eines zu untersuchenden Mittel unter Bedingungen, die für die Bindung des Liganden an die extrazelluläre Domäne des *daf*-4 Rezeptors geeignet sind und Bestimmen des Ausmaßes der Aktivität des Liganden zu der extrazellulären Domäne des *daf*-4 Rezeptors; und
(b) Vergleichen des Ausmaßes der in Schritt (a) bestimmten Bindung mit dem Ausmaß der Aktivität in Abwesenheit des zu untersuchenden Mittels, unter Bedingungen, die für die Bindung von Knochenmorphogenetischem Protein an die extrazelluläre Domäne des *daf*-4 Rezeptors geeignet sind,
wobei ein Unterschied in dem Ausmaß der in Schritt (a) bestimmten Bindung zu der in Schritt (b) bestimmten Bindung anzeigt, dass das Mittel die Aktivität von Knochenmorphogenetischem Protein-7 moduliert.

7. Verfahren nach Anspruch 6, worin der Ligand markiert ist, beispielsweise mit einem Radioisotop.

8. Verfahren nach Anspruch 6, worin das Knochenmorphogenetische Protein ausgewählt ist aus der Gruppe bestehend aus Knochenmorphogenetischem Protein-2, Knochenmorphogenetischem Protein-4, Knochenmorphogenetischem Protein-5, Knochenmorphogenetischem Protein-6, Activin und Knochenmorphogenetischem Protein-7.

9. Verfahren nach Anspruch 7, worin der Rezeptor ein Polypeptid mit der Aminosäuresequenz von SEQ ID NO. 1 oder SEQ ID NO. 2 umfasst.

10. Verfahren zum Identifizieren eines Mittels, welches die Aktivität von Knochenmorphogenetischem Protein-7 moduliert, umfassend:
(a) Kombinieren von radiomarkiertem Knochenmorphogenetischem Protein-7, der extarzellulären Domäne von *daf*-4 und einem Testmittel unter Bedingungen die für die Bindung von Knochenmorphogenetischem Protein an die extrazelluläre Domäne von daf-4 geeignet sind und Bestimmen des Ausmaßes der Bindung von Knochenmorphogenetischem Protein-7 an die extrazelluläre Domäne von *daf*-4; und
(b) Vergleichen des Ausmaßes der in (a) bestimmten Bindung mit dem Ausmaß der Bindung in Abwesenheit des Testmittels unter Bedingungen, die für die Bindung von Knochenmorphogenetischem Protein an die extrazelluläre Domäne des *daf*-4 Rezeptors geeignet sind;
wobei ein Unterschied in dem Ausmaß der Bindung in (a) zu der in (b) bestimmten Bindung anzeigt, dass das Mittel die Aktivität von Knochenmorphogenetischem Protein-7 moduliert.

11. Verfahren nach Anspruch 10, worin die extrazelluläre Domäne die Aminosäuresequenz von SEQ ID NO. 1 oder 2 aufweist.

12. Verfahren nach Anspruch 10, worin die extrazelluläre Domäne an einem festen Träger oder einer PVDF-Membran immobilisiert ist.

## Revendications

1. Procédé pour identifier un agent qui module l'activité de la protéine morphogénétique osseuse 7, ledit procédé comprenant les étapes consistant :
(a) à combiner un ligand de récepteur de protéine morphogénétique osseuse, un récepteur de type A pour la protéine morphogénétique osseuse et un agent à tester dans des conditions convenables pour la liaison du ligand au récepteur et à déterminer le degré de liaison du ligand au récepteur ; et
(b) à comparer le degré de liaison déterminé dans l'étape (a) avec le degré de liaison en l'absence de l'agent à tester dans des conditions convenables pour la liaison du ligand au récepteur ;
dans lequel une différence de degré de liaison déterminé dans l'étape (a) par rapport à la liaison déterminée dans l'étape (b) indique que l'agent module l'activité de la protéine morphogénétique osseuse 7.

2. Procédé suivant la revendication 1, dans lequel le ligand est marqué, par exemple avec un radioisotope.

3. Procédé suivant la revendication 1, dans lequel la protéine morphogénétique osseuse est choisie dans le groupe consistant en la protéine morphogénétique osseuse 2, la protéine morphogénétique osseuse 4, la protéine morphogénétique osseuse 5, la protéine morphogénétique osseuse 6, une activine et la protéine morphogénétique osseuse 7.

4. Procédé suivant la revendication 1, dans lequel le récepteur est le récepteur *daf*-4.

5. Procédé pour identifier un agent qui module l'activité de la protéine morphogénétique osseuse 7, ledit procédé comprenant les étapes consistant :
(a) à combiner un ligand de récepteur de protéine morphogénétique osseuse, le domaine extracellulaire d'un récepteur de type II de protéine morphogénétique osseuse et un agent à tester, dans des conditions convenables pour la liaison du ligand au domaine extracellulaire du récepteur, et à déterminer le degré de liaison du ligand au domaine extracellulaire du récepteur ; et
(b) à comparer le degré de liaison déterminé dans l'étape (a) avec le degré d'activité en l'absence de l'agent à tester dans des conditions convenables pour la liaison de la protéine morphogénétique osseuse au domaine extracellulaire du récepteur ;
dans lequel une différence de degré de liaison déterminé dans l'étape (a) par rapport à la liaison déterminée dans l'étape (b) indique que l'agent module l'activité de la protéine morphogénétique osseuse 7.

6. Procédé pour identifier un agent qui module l'activité de la protéine morphogénétique osseuse 7, ledit procédé comprenant les étapes consistant :
(a) à combiner un ligand de récepteur de protéine morphogénétique osseuse, le domaine extracellulaire du récepteur *daf*-4 et un agent à tester, dans des conditions convenables pour la liaison du ligand au domaine extracellulaire du récepteur *daf*-4, et à déterminer le degré d'activité du ligand au domaine extracellulaire du récepteur *daf*-4 ; et
(b) à comparer le degré de liaison déterminé dans l'étape (a) avec le degré d'activité en l'absence de l'agent à tester dans des conditions convenables pour la liaison de la protéine morphogénétique osseuse au domaine extracellulaire du récepteur *daf*-4 ;
dans lequel une différence de degré de liaison déterminé dans l'étape (a) par rapport à la liaison déterminée dans l'étape (b) indique que l'agent module l'activité de la protéine morphogénétique osseuse 7.

7. Procédé suivant la revendication 6, dans lequel le ligand est marqué, par exemple avec un radioisotope.

8. Procédé suivant la revendication 6, dans lequel la protéine morphogénétique osseuse est choisie dans le groupe consistant en : la protéine morphogénétique osseuse 2, la protéine morphogénétique osseuse 4, la protéine morphogénétique 5, la protéine morphogénétique 6, l'activine et la protéine morphogénétique osseuse 7.

9. Procédé suivant la revendication 7, dans lequel le récepteur comprend un polypeptide ayant la séquence d'aminoacides de la SEQ ID N° 1 ou de la SEQ ID N° 2.

10. Procédé pour identifier un agent qui module l'activité de la protéine morphogénétique osseuse 7, comprenant les étapes consistant :
(a) à combiner la protéine morphogénétique osseuse 7 radiomarquée, le domaine extracellulaire de *daf*-4 et un agent d'essai, dans des conditions convenables pour la liaison de la protéine morphogénétique osseuse au domaine extracellulaire de *daf-4,* et à déterminer le degré de liaison de la protéine morphogénétique osseuse 7 au domaine extracellulaire de *daf*-4 ; et
(b) à comparer le degré de liaison déterminé dans l'étape (a) avec le degré de liaison en l'absence de l'agent d'essai, dans des conditions convenables pour la liaison de la protéine morphogénétique osseuse au domaine extracellulaire de *daf*-4 ;
dans lequel une différence de degré de liaison en (a) par rapport à la liaison déterminée en (b) indique que l'agent module l'activité de la protéine morphogénétique osseuse 7.

11. Procédé suivant la revendication 10, dans lequel le domaine extracellulaire a la séquence d'aminoacides de la SEQ ID N° 1 ou 2.

12. Procédé suivant la revendication 10, dans lequel le domaine extracellulaire est immobilisé sur un support solide ou une membrane en PVDF.
